# EUROPEAN PATENT SPECIFICATION

(11) **EP 1 243 261 B1**
(45) Date of publication and mention of the grant of the patent: **27.04.2005**
(21) Application number: 02003106.8
(22) Date of filing: 13.02.2002
(51) Int. Cl.: A61K 31/00, A61K 31/4166, A61P 7/00

(54) **Use of a hydantoin derivative in a pharmaceutical composition against hypoalbuminaemia**
Verwendung von Hydantoin und seinen Derivaten gegen Hypalbuminämie
Utilisation de la hydantoine et de ses dérivées contre l'hypoalbuminémie

(30) Priority: 13.02.2001 JP 2001035262
(43) Date of publication of application: 25.09.2002
(73) Proprietor: NIPPON ZOKI PHARMACEUTICAL CO., LTD., Chuo-ku, Osaka (JP)
(72) Inventor: Ienaga, Kazuharu, 1-2, Hiranomachi Nichome, Chuo-ku, Osaka (JP); Mikami, Hiroki, Institute of Bio-Active Science, Yashiro-cho, Katoh-gun, Hyogo (JP); Nishibata, Ryoji, Institute of Bio-Active Science, Yashiro-cho, Katoh-gun, Hyogo (JP)
(74) Representative: HOFFMANN - EITLE

(56) References cited:
- EP-A- 0 412 940
- EP-A- 0 444 546
- EP-A- 0 640 594
- WO-A-01/56996
- FR-A- 2 796 945
- US-A- 5 202 339
- BOSTON COLLAB DRUG SURV PROGRAM: "DI PHENYL HYDANTOIN SIDE EFFECTS AND SERUM ALBUMIN LEVELS" CLINICAL PHARMACOLOGY & THERAPEUTICS, vol. 14, no. (4 PART 1, 1973, pages 529-532, XP008006157 ISSN: 0009-9236
- ALLEN J K ET AL: "EFFECTS OF DI PHENYL HYDANTOIN ON THE RELATIONSHIP BETWEEN HIGH DENSITY LIPO PROTEIN CHOLESTEROL AND SEVERAL BIOCHEMICAL ASSAYS" CLINICA CHIMICA ACTA, vol. 102, no. 1, 1980, pages 111-114, XP008006158 ISSN: 0009-8981

## Description

### Technical Field of the Invention

The present invention relates to the use of a hydantoin derivative or a pharmaceutically acceptable salt, hydrate or complex thereof as an effective ingredient in the manufacture of a medicament effective in the treatment of hypoalbuminaemia.

### Prior Art

The concentration of serum albumin in a healthy subject is 35-55 mg/ml. A concentration of < 35 mg/ml is diagnosed as hypoalbuminaemia, and a serum albumin concentration of < 30 mg/ml is a condition of serious hypoalbuminaemia with clinical relevance. Hypoalbuminaemia causes symptoms such as edema and ascites, and worsens disorders by hyperbilirubinemia.

Hypoalbuminaemia is caused by various dysfunctions such as decrease of albumin synthesis, loss of albumin from the blood, and dilution of plasma. Albumin is synthesized only by hepatocytes. The main causes of a decreased synthesis are a deficient amino acid supply due to nutrition disorders and a lowered ability to synthesize albumin due hepatic diseases. The loss of albumin from blood is caused by renal failure such as nephritic syndrome, traumatic injury such as burn and protein-losing gastroenteropathy. Dilution of plasma may, among others, be caused by heart failure or accelerated catabolism due to hyperthyroidism.

Heretofore hypoalbuminaemia was treated by a dietetic therapy using high protein diet and/or a drug therapy using total amino acid preparations. However, the intravenous administration of amino acids requires high caution due to potential side effects such as acid-base balance disorders, hyperammonaemia, imbalance of amino acids and azotemia. Also, if blood protein preparations are used, a problem exists in that such preparations may be contaminated by e.g. viruses.

As mentioned above, at present mainly amino acid preparations and blood preparations for intravenous administration are used in the therapy of hypoalbuminaemia. There is a brisk demand in clinical fields for a therapeutic agent providing a having higher safety and which is available for oral administration.

The compounds referred to herein are known to have plant growth controlling action. Also, they are known to have pharmacological actions such as hypoglycemic and hypolipemic actions, and also exhibit low toxicity resulting in almost no side effects (see e.g. Japanese Patent Application Publications Sho-57/114578, Sho-60/188373, Sho-61/122275, Sho-62/45525, Sho-62/14, Hei-01/75473 and Hei-01/299276). It has been also disclosed that the compounds are useful as a uremic toxin-lowering agent (Japanese Patent Application Publication Hei-03/72463), as an agent eliminating active oxygen and free radicals (Japanese Laid-Open Patent Publication Hei-09/227377) and as a therapeutic agent for intractable vasculitis (Japanese Patent Application Publication 2000/212083). However, any therapeutic effect of the present compounds against hypoalbuminaemia has not yet been discovered.

EP-A-0 412 940 discloses the use of hydantoin or a derivative, a pharmaceutically acceptable salt or a complex thereof as an active agent useful in the prevention or treatment of renal failure in a mammal.

FR-2 796 945 describes a broad group of chemical compounds of a general formula (I), including hydantoin derivatives carrying an imidazol-2-yl or imidazol-2-yl alkyl group as a substituent. These groups are not encompassed within the definition of the substituents X or Y of the compounds referred to in the present invention. It is further described in FR-2 796 945 that these compounds are effective as pharmaceuticals in the treatment of many different diseases, which however, do not include the disease of concern in the present application.

EP-A-0 640 594 describes compounds including hydantoin derivatives as metalloprotease inhibitors having an inhibitory activity on neutral metalloendopeptidase (NEP) or angiotensin converting enzyme (ACE). They are said to be useful as drugs in the treatment of cardiovascular diseases, such as hypertension and heart failure, as well as analgesic drugs. The hydantoin derivatives included within the disclosure of EP-A-0 640 594 have in the 3-position of the ring a substituent group R¹S-A-CR²R³-, wherein A is an optionally substituted divalent aliphatic C₁₋₁₀-hydrocarbon group.

EP-A-0 444 546 describes hydantoin derivatives which are useful as hypoglycaemic and/or hypolipidemic agents. The compounds of concern are hydantoin derivatives having in the 1-position of the hydantoin ring a group Q-SO₂-, wherein Q may be selected from a broad variety of optionally substituted alkyl, cycloalkyl, biphenylyl and heterocyclic groups.

US 5,202,339, claims the same priority as EP-A-0 444 546 discussed above, discloses basically the same hydantoin derivatives as the EP-application document. The compounds are described to be useful in treatment and/or prevention of various forms of diabetic complications, such as neuropathy, autonomic disease, cataracts, retinopathy, nephropathy, and microvascular disease.

In an article of the Boston Collaborative Drug Surveillance Program, Clinical Pharmacology and Therapeutics, 14(4) part 1, 529-532 (1973), diphenylhydantoin side effects in serum albumin levels are described. In a clinical trial involving 322 patients, it has been found that upon administration of diphenylhydantoin the frequency of adverse reactions in patients having low albumin levels was higher by a factor of 3.2 as compared with patients having normal albumin levels. Hence, as a result the study revealed that in patients with impaired renal function, the risk of diphenylhydantoin toxicity is increased.

### Summary of the Invention

In view of the above an object of the present invention is to solve the problems connected with known therapeutic agents against hypoalbuminaemia, and is to offer a therapeutic agent for hypoalbuminaemia having a higher safety and which is available for oral administration.

The present inventors have carried out intensive investigations and have found that a composition comprising at least one compound of the formula (I) 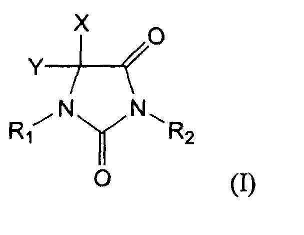 wherein R₁ and R₂ each independently is H, C₁₋₂₀-alkyl or C₃₋₈-cycloalkyl; and X and Y each independently is H, OH, C₁₋₃-alkyl or C₁₋₅-alkoxy, or X and Y together form an oxo group, or a pharmaceutically acceptable salt, hydrate or complex thereof, has a therapeutic effect against hypoalbuminaemia upon oral administration, while not being toxic and showing almost no side effects, whereupon the present invention has been accomplished.

### Detailed Description

The effective ingredient used in the manufacture of a pharmaceutical composition of the present invention is a hydantoin derivative of the following formula (I). 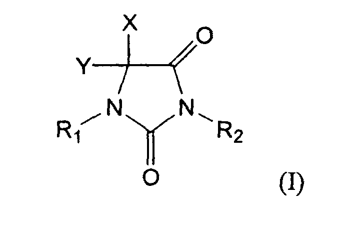 wherein R₁ and R₂ each independently is H, C₁₋₂₀-alkyl or C₃₋₈-cycloalkyl; and X and Y each independently is H, OH, C₁₋₃-alkyl or C₁₋₅-alkoxy, or X and Y together form an oxo group, or a pharmaceutically acceptable salt, hydrate or complex thereof.

In formula (I), R₁ and R₂ each independently represent H, a straight or branched C₁₋₂₀-alkyl group such as methyl, ethyl, propyl, isopropyl, butyl, isobutyl, sec-butyl, t-butyl, pentyl, isopentyl, neopentyl, t-pentyl, hexyl, dimethylbutyl, heptyl, octyl, nonyl, decyl or stearyl, or a C₃₋₈-cycloalkyl group such as cyclopropyl, cyclobutyl, cyclopentyl, cyclohexyl, cycloheptyl or cyclooctyl.

X and Y each independently represent H, OH group, a straight or branched C₁₋₃-alkyl such as methyl, ethyl, propyl or isopropyl, or a straight or branched C₁₋₅-alkoxy such as methoxy, ethoxy, propoxy, isopropoxy, butoxy, isobutoxy, sec-butoxy, t-butoxy, pentoxy, isopentoxy or neopentoxy, or X and Y together represent an oxo group.

Preferred compounds of the present invention are listed as follows:
- [1]: Hydantoin
- [2]: 1-Methyl-hydantoin
- [3]: 3-Methyl-hydantoin
- [4]: 1-Ethyl-hydantoin
- [5]: 1-Propyl-hydantoin
- [6]: 1-Butyl-hydantoin
- [7]: 1-t-Butyl-hydantoin
- [8]: 1-Hexyl-hydantoin
- [9]: 1-(1,3-Dimethylbutyl)-hydantoin
- [10]: 1-Decyl-hydantoin
- [11]: 1-Stearyl-hydantoin
- [12]: 1,3-Dimethyl-hydantoin
- [13]: 1,5-Dimethyl-hydantoin
- [14]: 3,5-Dimethyl-hydantoin
- [15]: 1-Cyclopentyl-hydantoin
- [16]: 1-Cyclohexyl-hydantoin
- [17]: 1-Cyclohexyl-3-methyl-hydantoin
- [18]: 3-Cyclohexyl-hydantoin
- [19]: 1,3-Dicyclohexyl-hydantoin
- [20]: 5-Hydroxy-hydantoin
- [21]: 5-Hydroxy-1-methyl-hydantoin
- [22]: 5-Hydroxy-3-methyl-hydantoin
- [23]: 5-Hydroxy-1-ethyl-hydantoin
- [24]: 5-Hydroxy-1-propyl-hydantoin
- [25]: 5-Hydroxy-1-butyl-hydantoin
- [26]: 5-Hydroxy-1-t-butyl-hydantoin
- [27]: 5-Hydroxy-1-hexyl-hydantoin
- [28]: 5-Hydroxy-1-(1,3-dimethylbutyl)-hydantoin
- [29]: 5-Hydroxy-1-decyl-hydantoin
- [30]: 5-Hydroxy-1-stearyl-hydantoin
- [31]: 5-Hydroxy-1-cyclopentyl-hydantoin
- [32]: 5-Hydroxy-1-cyclohexyl-hydantoin
- [33]: 5-Hydroxy-1-cyclohexyl-3-methyl-hydantoin
- [34]: 5-Hydroxy-1,3-dimethyl-hydantoin
- [35]: 5-Hydroxy-1,5-dimethyl-hydantoin
- [36]: 5-Hydroxy-3,5-dimethyl-hydantoin
- [37]: 5-Hydroxy-1,3-dicyclohexyl-hydantoin
- [38]: 5-Methoxy-hydantoin
- [39]: 5-Methoxy-1-methyl-hydantoin
- [40]: 5-Methoxy-3-methyl-hydantoin
- [41]: 5-Methoxy-1-ethyl-hydantoin
- [42]: 5-Methoxy-1-propyl-hydantoin
- [43]: 5-Methoxy-1-butyl-hydantoin
- [44]: 5-Methoxy-1-cyclohexyl-hydantoin
- [45]: 5-Methoxy-3-cyclohexyl-hydantoin
- [46]: 5-Ethoxy-hydantoin
- [47]: 5-Ethoxy-1-methyl-hydantoin
- [48]: 5-Ethoxy-3-methyl-hydantoin
- [49]: 5-Ethoxy-1-ethyl-hydantoin
- [50]: 5-Ethoxy-1-propyl-hydantoin
- [51]: 5-Ethoxy-1-butyl-hydantoin
- [52]: 5-Propoxy-hydantoin
- [53]: 5-Propoxy-1-methyl-hydantoin
- [54]: 5-Propoxy-3-methyl-hydantoin
- [55]: 5-Propoxy-1-ethyl-hydantoin
- [56]: 5-Propoxy-1-propyl-hydantoin
- [57]: 5-Propoxy-1-butyl-hydantoin
- [58]: 5-Butoxy-hydantoin
- [59]: 5-Butoxy-1-methyl-hydantoin
- [60]: 5-Butoxy-3-methyl-hydantoin
- [61]: 5-t-Butoxy-hydantoin
- [62]: 5-t-Butoxy-1-methyl-hydantoin
- [63]: 5-t-Butoxy-3-butyl-hydantoin
- [64]: Imidazolidinetrione
- [65]: 1-Methyl-imidazolidinetrione
- [66]: 1-Ethyl-imidazolidinetrione
- [67]: 1-Butyl-imidazolidinetrione
- [68]: 1-Isobutyl-imidazolidinetrione
- [69]: 1-t-Butyl-imidazolidinetrione
- [70]: 1-Hexyl-imidazolidinetrione
- [71]: 1-(1,3-Dimethylbutyl)-imidazolidinetrione
- [72]: 1-Decyl-imidazolidinetrione
- [73]: 1-Cyclopentyl-imidazolidinetrione
- [74]: 1-Cyclopentyl-3-ethyl-imidazolidinetrione
- [75]: 1-Cyclohexyl-imidazolidinetrione
- [76]: 1,3-Dimethyl-imidazolidinetrione
- [77]: 1,3-Dicyclohexyl-imidazolidinetrione

The hydantoin derivatives encompassed within the present invention include the pharmaceutically acceptable salts of the compounds of formula (I), preferably acid addition salts with hydrochloric, sulfuric, nitric, hydrobromic, phosphoric, perchloric, thiocyanic, boric, formic, acetic, haloacetic, propionic, glycolic, citric, tartaric, succinic, gluconic, lactic, malonic, fumaric, anthranilic, benzoic, cinnamic, p-toluenesulfonic, naphthalenesulfonic or sulfanilic acid; or salts with alkali metals (preferably Na or K), alkaline-earth metals (preferably Ca, Mg or Ba) or other metals, such as Al or Zn.

Those salts may be manufactured by known methods from the respective free hydantoin derivatives or may be obtained by converting one salt into another.

If steric isomers, such as cis-trans, optical or conformational isomers of the substances of formula (I) are possible, they are all included within the useful effective agent of the pharmaceutical composition of the present invention.

The compounds of formula (I) and methods for manufacturing them are disclosed in e.g. Japanese Laid Open (Kokai) Nos. 61/122275 and 62/14.

The compounds of formula (I) can be made into pharmaceutical preparations by combining them with one or more suitable pharmaceutical carriers or diluents, optionally together with one or more additives which are commonly used in the field of pharmaceutical compositions, such as fillers, antioxidants, disintegration agents, binders, flavors, pigments, colorants, coatings, lubricants, etc..

The pharmaceutical composition may be present in any form for known methods of administration, i.e. for oral administration (e.g. tablets, capsules, powders, liquids, etc.) and for parenteral administration (e.g. for subcutaneous, intravenous, intramuscular, intrarectal and intranasal administrations). Any known process for producing such dosage forms may be used to produce the pharmaceutical compositions of the present invention.

To prepare the pharmaceutical compositions, the compounds of formula (I) may be used in their free form, or as a pharmaceutically acceptable salt, hydrate or complex thereof. Also, one or more compound(s) of Formula (I) may be present in a pharmaceutical composition as the only active principle, or together with other pharmaceutically active ingredients.

In the case of preparations for oral administration, the active compound(s), optionally together with one or more commonly used excipients is mixed with one or more binders and optionally other additives, and is then processed into oral formulations such as tablets, powders, granules or capsules.

Suitable excipients are, for example, lactose, mannitol, corn starch, potato starch or potassium citrate. Examples for suitable binders are cellulose derivatives (e.g. crystalline cellulose or hydroxypropylcellulose), gum arabicum, corn starch or gelatin. Furthermore, examples for suitable disintegration agents are corn starch, potato starch, calcium carboxymethylcellulose. Other optional additives may be exemplifies as lubricating agents such as talc or magnesium stearate, bulking agents, moisturizing agents, buffers, preservatives, perfumes, etc.

In the case of preparations for injections, the active principle, optionally together with suitable, commonly known additives, can be processed into a solution or suspension in aqueous or non-aqueous solvents, such as distilled water for injection, physiological saline solution, Ringer's solution, plant oil, synthetic fatty acid glycerides, higher fatty acid esters or propylene glycol.

If desired, it is also possible to prepare pharmaceutical preparations for other administration pathways, such as syrups, suppositories, inhalations, aerosol preparations, collyriums or medicines for external use (e.g. ointments, gels, poultices).

The preferred dose of the effective substance of the pharmaceutical compositions may vary, depending on the sex, age, condition etc. of the patient and/or the specific nature of the disease to be treated, the form of the preparation, the method and/or the term of administration.

In general, to achieve a desired effect, 1-1,000 mg/day, preferably 5-600 mg/day, may be given to common adults by oral route. Due to the influence of e.g. the different absorption properties, in the case of a parenteral administration such as by injection, it is preferred that the daily dose is at a level of from 1/3 to 1/10 of the usual oral dose given above, i.e. preferably the daily dose is 0.1-333 mg/day, more preferably 0.5-200 mg/day.

Further preferred embodiments of pharmaceutical compositions prepared in accordance with the present invention comprising at least one compound of the above formula (I) are as defined in the appended claims 2-7.

The hypoalbuminaemia against which the pharmaceutical compositions comprising a hydantoin derivative as described herein are effective may have a broad variety of causes, i.e. the hypoalbuminaemia may be the result of various underlying diseases or dysfunctions. For example, the pharmaceutical compositions are effective in the treatment of hypoalbuminaemia caused by hepatic diseases, nutrition disorders, renal failure, traumatic injury, protein-losing gastroenteropathy, heart failure or hypercatabolism.

The present invention is further illustrated by the following example.

### [Examples]

### Example 1. The result of clinical test

5-Hydroxy-1-methyl-hydantoin (Compound 21 listed above) was administered in an amount of 200 mg/day (Patient 1: 400 mg/day) to 6 patients suffering from hypoalbuminaemia (serum albumin value was < 30 mg/ml). Serum albumin values were taken before the treatment, and after administration for 24 weeks (Patient 2: 16 weeks). The results are shown in Table 1 (the significant of the differences was statistically analyzed by a paired t-test).

**Table 1.**

| | Before administration | After administration for 24 weeks |
|---|---|---|
| Patient 1 | 1.7 g/dL | 2.1 g/dL |
| Patient 2 | 2.0 g/dL | 2.3¹⁾ g/dL |
| Patient 3 | 2.3 g/dL | 3.4 g/dL |
| Patient 4 | 2.4 g/dL | 2.7 g/dL |
| Patient 5 | 2.5 g/dL | 3.2 g/dL |
| Patient 6 | 2.7 g/dL | 3.0 g/dL |
| Mean Value | 2.3±0.2 g/dL | 2.8±0.2²⁾ g/dL |

| | | |
|---|---|---|
| ¹⁾ after 16 weeks | | |
| ²⁾ p < 0.01 | | |

The serum albumin values were significantly increased by the administration of the above pharmaceutical composition, thus showing the efficacy thereof in the treatment of hypoalbuminaemia. When the same test as above was performed with healthy persons having a normal serum albumin value, no changes of this value was observed.

It is apparent from the clinical test that a pharmaceutical composition prepared in accordance with the present invention is very useful therapeutic agent against hypoalbuminaemia. Since no effect is observed in subjects with normal serum albumin values, it is considered that the pharmaceutical composition yields an effect only in morbid states of abnormally lowered serum albumin values.

In addition, it has been shown in animal as well as in clinical tests that the pharmaceutical composition has little to no side effects. Thus, since the pharmaceutical composition shows a good therapeutic effect together with a high safety, it is very useful as a drug for oral and/or long-term treatment of hypoalbuminaemia.

## Claims

1. Use of at least one hydantoin derivative of the formula (I) 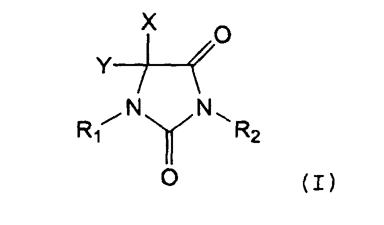 wherein R₁ and R₂ each independently is H, C₁₋₂₀-alkyl or C₃₋₈-cycloalkyl; and X and Y each independently is H, OH, C₁₋₃-alkyl or C₁₋₅-alkoxy, or X and Y together form an oxo group, or a pharmaceutically acceptable salt, hydrate or complex thereof, for the manufacture of a medicament effective in the treatment of hypoalbuminaemia.

2. Use of claim 1, wherein in formula (I) X is H and Y is OH.

3. Use of claim 1 or 2, wherein one of R₁ and R₂ is H and the other one is a C₁₋₂₀-alkyl group.

4. Use of any of claims 1-3, wherein R₁ is a C₁₋₂₀-alkyl group.

5. Use of claim 4, wherein R₁ is a C₁₋₄ alkyl group.

6. Use of claim 5, wherein R₁ is methyl.

7. Use of any of the preceding claims, wherein the compound of formula (I) is 5-hydroxy-1-methylhydantoin or a pharmaceutically acceptable salt thereof.

8. Use of any of the preceding claims, wherein the hypoalbuminaemia is caused by at least one of hepatic disease, nutrition disorders, renal failure, traumatic injury, protein-losing gastroenteropathy, heart failure and hypercatabolism.

## Patentansprüche

1. Verwendung mindestens eines Hydantoinderivats der Formel (I): 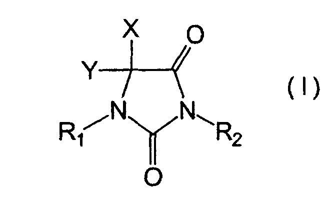 worin R₁ und R₂ jeweils unabhängig voneinander H, C₁₋₂₀-Alkyl oder C₃₋₈-Cycloalkyl darstellen; und X und Y sind jeweils unabhängig voneinander H, OH, C₁₋₃-Alkyl oder C₁₋₅-Alkoxy, oder X und Y bilden zusammen eine Oxogruppe, oder ein pharmazeutisch annehmbare(s/r) Salz, Hydrat oder Komplex davon, zur Herstellung eines Medikaments, das bei der Behandlung von Hyperalbuminämie wirksam ist.

2. Verwendung gemäss Anspruch 1, worin in Formel (I) X H und Y OH ist.

3. Verwendung gemäss Anspruch 1 oder 2, worin eines von R₁ und R₂ H ist und das andere ist eine C₁₋₂₀-Alkylgruppe.

4. Verwendung gemäss mindestens einem der Ansprüche 1 bis 3, worin R₁ eine C₁₋₂₀-Alkylgruppe ist.

5. Verwendung gemäss Anspruch 4, worin R₁ eine C₁₋₄-Alkylgruppe ist.

6. Verwendung gemäss Anspruch 5, worin R₁ Methyl ist.

7. Verwendung gemäss mindestens einem der vorhergehenden Ansprüche, worin die Verbindung der Formel (I) 5-Hydroxy-1-methylhydantoin oder ein pharmazeutisch annehmbares Salz davon ist.

8. Verwendung gemäss mindestens einem der vorhergehenden Ansprüche, worin die Hyperalbuminämie hervorgerufen wird durch mindestens eines aus Lebererkrankungen, Ernährungsstörungen, Nierenversagen, traumatischen Verletzungen, Gastroenteropathie mit Proteinverlust, Herzinsuffizienz und Hyperkatabolismus.

## Revendications

1. Utilisation d'au moins un dérivé d'hydantoïne de formule (I) 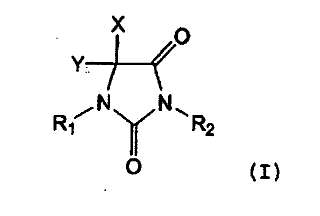 dans laquelle R₁ et R₂ représentent chacun indépendamment H, un groupe alkyle en C₁-C₂₀ ou cycloalkyle en C₃-C₈, X et Y représentant chacun indépendamment H, OH, un groupe alkyle en C₁-C₃ ou alcoxy en C₁-C₅ ou X et Y formant ensemble un groupe oxo,
ou de son sel, hydrate ou complexe pharmaceutiquement acceptable, pour la préparation d'un médicament efficace dans le traitement de l'hypoalbuminémie.

2. Utilisation selon la revendication 1, dans laquelle X représente H et Y représente OH dans la formule (I).

3. Utilisation selon la revendication 1 ou 2, dans laquelle l'un parmi R₁ et R₂ représente H, l'autre représentant un groupe alkyle en C₁-C₂₀.

4. Utilisation selon l'une quelconque des revendications 1 à 3, dans laquelle R₁ représente un groupe alkyle en C₁-C₂₀.

5. Utilisation selon la revendication 4, dans laquelle R₁ représente un groupe alkyle en C₁-C₄.

6. Utilisation selon la revendication 5, dans laquelle R₁ représente un groupe méthyle.

7. Utilisation selon l'une quelconque des revendications précédentes, dans laquelle le composé de formule (I) est la 5-hydroxy-1-méthylhydantoïne ou son sel pharmaceutiquement acceptable.

8. Utilisation selon l'une quelconque des revendications précédentes, dans laquelle l'hypoalbuminémie est provoquée par au moins l'une des affections que sont les maladies hépatiques, les troubles nutritionnels, l'insuffisance rénale, une lésion traumatique, la gastroentéropathie avec perte de protéines, l'insuffisance cardiaque et l'hypercatabolisme.
